(19) **Europäisches Patentamt**
**European Patent Office**
**Office européen des brevets**

(11) **EP 4 397 254 A1**

(12) **EUROPEAN PATENT APPLICATION**
published in accordance with Art. 153(4) EPC

(43) Date of publication:
**10.07.2024  Bulletin 2024/28**

(51) International Patent Classification (IPC):
***A61B 17/22*** (2006.01)   ***A61B 5/24*** (2021.01)
***G01R 19/165*** (2006.01)

(21) Application number: **22863480.4**

(22) Date of filing: **30.08.2022**

(86) International application number:
**PCT/CN2022/115995**

(87) International publication number:
**WO 2023/030345 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **30.08.2021  CN 202111005958**

(71) Applicant: **Jiangsu Polylive Medtech Co., Ltd.**
**Nantong, Jiangsu 226400 (CN)**

(72) Inventors:
• **LI, Chuang**
 **Nantong, Jiangsu 226400 (CN)**
• **TANG, Zhihuang**
 **Nantong, Jiangsu 226400 (CN)**

(74) Representative: **Zaboliene, Reda**
**Metida**
**Business center Vertas**
**Gyneju str. 16**
**01109 Vilnius (LT)**

(54) **CURRENT PEAK VALUE MEASUREMENT APPARATUS, HIGH VOLTAGE GENERATOR, AND THERAPEUTIC DEVICE FOR VASCULAR CALCIFICATION**

(57)    A current peak value measurement apparatus (100), a high voltage generator (200), and a therapeutic device for vascular calcification. The current peak value measurement apparatus (100) comprises a PCB Rogowski coil (10), a sampling resistor (11), a signal amplification circuit (12), an integration and voltage retaining circuit (13) and a micro-controller (14), wherein the PCB Rogowski coil (10) is used for sensing a current signal in a line to be tested, and for obtaining a corresponding induced electromotive force; the sampling resistor (11) is connected to the PCB Rogowski coil (10); the signal amplification circuit (12) is connected to the sampling resistor (11); the integration and voltage retaining circuit (13) is connected to the signal amplification circuit (12); and the micro-controller (14) is connected to the integration and voltage retaining circuit (13), and is used for performing ADC sampling on the maximum value of a voltage signal, and for obtaining a current peak value of the current signal in the line to be tested according to ADC sampling data and preset current rating data. The apparatus (100) can realize real-time monitoring over a pulse current and accurately measure a current peak value of the pulse current, which does not affect an original high-voltage discharge loop, and the apparatus has the advantages of a high safety, a fast response speed, a stable process, a high consistency and a low cost.

**EP 4 397 254 A1**

FIG. 1

**Description**

The present application claims priority to a Chinese patent application No. CN202111005958.6, filed on August 30, 2021, entitled "CURRENT PEAK VALUE MEASUREMENT APPARATUS, HIGH VOLTAGE GENERATOR, AND THERAPEUTIC DEVICE FOR VASCULAR CALCIFICATION", the entire contents of which are incorporated by reference in the present application.

TECHICAL FIELD

The present invention relates to the field of current detection technology, and is especially a current peak value measurement apparatus, a high voltage generator and a therapeutic device for vascular calcification.

BACKGROUND

Vascular calcification refers to the pathological process of hydroxyapatite mineral deposition in the vascular system, which is commonly seen in atherosclerotic plaques, diabetes, aging, chronic renal failure, uremia, and blood vessels and heart valves. Clinical and epidemiological data show that vascular calcification is a common clinical pathological feature in patients with atherosclerosis, hypertension, diabetic vascular disease, vascular injury and chronic kidney disease. It is an important risk factor leading to cardiovascular events, occurring in 80% of vascular injury and 90% of coronary disease. Vascular calcification in infancy, uremic arteriolar calcification, and calcified valvular disease can be fife-threatening. The incidence of vascular calcification in young patients is more common in diabetic patients, while dyslipidemia is more common in elderly patients. In recent years, studies on vascular biology and cardiovascular imaging have found that the location and degree of vascular calcification can be used as an early warning indicator for stroke and ischemic heart disease, and an indicator for predicting cardiovascular mortality in patients with type II insulin-independent diabetes mellitus. Vascular calcification was previously thought to be a passive process of calcium salt deposition between tissues caused by the imbalance of calcium and phosphorus metabolism. Vascular calcification increases the stiffness of the vascular wall, reduces the compliance, and then leads to myocardial ischemia, left ventricular hypertrophy and heart failure, leading to thrombosis and plaque rupture. It is one of the important factors for the high morbidity and mortality of cardiovascular and cerebrovascular diseases, and is also an important marker molecule for the occurrence of cardiovascular and cerebrovascular diseases.

At present, the main treatment methods of vascular calcification are medical treatment and surgical treatment. Medical treatment is to use lipid-regulating drugs to stabilize the intravascular plaque and slow down the progression of atherosclerosis, or to regulate the overall calcium and phosphorus metabolism through phosphate binders, bisphosphonates and other calcium and phosphorus balance regulators to reduce calcium salt deposition and calcium crystallization nucleation. However, the effect of these drugs on vascular calcification is not clear, and the effect is poor in patients with advanced vascular calcification. Surgical treatment includes interventional therapy and arterial bypass grafting. Interventional devices include traditional balloon, special balloon and percutaneous transluminal plaque resection, but these devices and techniques still have many limitations and are prone to lead to serious complications. Traditional balloon dilatation only has a good effect on soft lesions, but in the treatment of calcified lesions, higher pressure is needed to restore the lumen again. However, continuous high pressure is easy to cause secondary mechanical damage to the blood vessels, and even cause vascular rupture. Special balloons, such as embedded balloons and spinous balloons, have special conditions for use and require longer training. Percutaneous atherectomy is expensive, complex and time-consuming, and it is difficult to solve the problem of distal embolism and high risk of vascular injury. In order to solve the above problems in the treatment of calcified vessels, the combination of ultrasonic lithotripsy and balloon angioplasty technology can better overcome the shortcomings of traditional device and technology in the treatment of calcified artery disease by using acoustic waves, and is expected to become a new generation of treatment technology for calcified vessels.

SUMMARY OF THE INVENTION

Technical Problem

The therapeutic device for vascular calcification is mainly composed of a consumable electrode, a connector, and an ultrasound generator. In the treatment of vascular calcification, the instantaneous current of electrode discharge is large, which can reach hundreds of amperes, and the discharge voltage is high, which can reach ten kilovolts. At present, there is no electrode current sampling device in the therapeutic device for vascular calcification, so it is impossible to capture the current peak value during treatment in real time, and it is impossible to confirm the operation of the device and the treatment of the patient, so as to further analyze the treatment results. The treatment effect cannot be monitored in real time, and the treatment status of the patient cannot be monitored during the treatment process and the treatment strategy can be adjusted in time. The treatment effect and safety of patients are difficult to be guaranteed.

The existing scheme of increasing the sampling resistance in the high-voltage circuit has an impact on the original discharge circuit, and there are security risks. The existing scheme of using external electrode current sampling device can be more convenient to expand in laboratory test and verification, but in actual use, it is difficult to be applied due to the high price of professional device and large volume of device, inconvenient to install and carry, difficult to implement and difficult to use in the field. In the existing current transformer scheme, due to the existence of magnetic components in the transformer, it is inevitable that there will be magnetic saturation, and the measured current under magnetic saturation does not have reference significance. At the same time, there will be a certain phase difference due to the hysteresis effect of ferromagnetic components.

Problem Solution

Technical Solution

In view of this, the object of the present invention is to provide a current peak value measurement apparatus, a high voltage generator and a therapeutic device for vascular calcification. The real-time monitoring of pulse current can be realized, the current peak value of the pulse current can be accurately detected, the original discharge loop cannot be influenced, and the present invention has the advantages of high safety, fast response speed, large saturation current, simple structure, stable process, high consistency and low cost.

In order to achieve the above object, a first aspect of an embodiment of the present invention provides a current peak value detecting device, as one embodiment, the current peak value detecting device includes: a PCB Rogowski coil, a sampling resistor, a signal amplifying circuit, an integrating and voltage holding circuit, and a micro-controller; wherein,

the PCB Rogowski coil is used for induce a current signal in a circuit to be detected, and obtain a corresponding induced electromotive force;
the sampling resistor is connected to the PCB Rogowski coil, and is configured to obtain a sampling voltage corresponding to the induced electromotive force;
the signal amplifying circuit is connected to the sampling resistor, and is configured to amplify the sampling voltage and output amplified sampling voltage;
the integrating and voltage holding circuit is connected to the signal amplifying circuit, and is configured to perform integrating processing on the amplified sampling voltage to obtain a voltage signal proportional to the current signal in the circuit to be detected, and to hold a maximum value of the voltage signal;
the micro-controller is connected to the integrating and voltage holding circuit and is used for carrying out ADC sampling on the maximum value of the voltage signal and obtaining a current peak value of the current signal in the circuit to be measured according to the ADC sampling data and preset current calibration data.

As one implementation mode, the PCB Rogowski coil includes a first coil part and a second coil part, the first coil part and the second coil part form an loop-shaped coil whole body, and the first coil part and the second coil part are detachably connected; wherein,
the first coil part and the second coil part are respectively provided with conducting wires on a top layer and a bottom layer correspondingly, the corresponding conducting wires are connected through via holes to form wire turns and are provided with return wires.

As one embodiment, the integrating and voltage holding circuit includes an RC integrating circuit and a first diode, the RC integrating circuit including a first resistor and a first capacitor; wherein,
the first diode and the first resistor are serially connected between the output of the signal amplifying circuit and the input terminal of the micro-controller, and the first capacitor is connected between the input terminal of the micro-controller and ground.

As one implementation manner, the micro-controller is further configured to perform digital filtering on the ADC sampling data to filter abnormal data in a plurality of the ADC sampling data, and take an average value of the plurality of the filtered ADC sampling data for obtaining a current peak value of the current signal in the circuit to be detected according to the current calibration data; wherein the abnormal data includes the minimum and/or the maximum of a plurality of the ADC sampled data.

As one implementation mode, the utility model also includes a filter circuit which is arranged between the PCB Rogowski coil and the signal amplifying circuit.

As one embodiment, the filter circuit includes a common-mode inductor arranged between an output terminal of the PCB Rogowski coil and the sampling resistor.

In one embodiment, the filter circuit includes a first magnetic bead and a second magnetic bead connected in parallel between the sampling resistor and the signal amplifying circuit.

In one embodiment, the filter circuit includes a common-mode filter capacitor and a differential-mode filter capacitor disposed between the sampling resistor and the signal amplification circuit.

In order to achieve the above object, another embodiment of the present invention provides a high voltage generator, as one embodiment, the high voltage generator includes a power supply module, a booster circuit and the current peak value measurement apparatus described in any one of the above embodiments. wherein,

the power supply module is connected to the booster circuit and the micro-controller in the current peak value measurement apparatus;

the micro-controller in the current peak value measurement apparatus, is connected to the booster circuit;

the PCB coil in the current peak value measurement apparatus is arranged at the output terminal of the booster circuit and is used for inducing the current signal in the circuit to be detected at the output terminal.

In order to achieve the above object, another embodiment of the present invention provides a therapeutic device for vascular calcification, as one embodiment, the therapeutic device for vascular calcification includes the high voltage generator, a connector and a consumable electrode of the above embodiment; wherein,

the output terminal of the high voltage generator is connected to the consumable electrode by means of the connector so as to form high-voltage discharge loop.

Beneficial Effect Of The Invention

Beneficial Effect

The present invention includes a current peak value measurement apparatus, a high voltage generator and a therapeutic device for vascular calcification, including a PCB Rogowski coil, a sampling resistor, a signal amplification circuit, an integration and voltage holding circuit and a micro-controller; wherein the PCB Rogowski coil is configured to induce the current signal in a circuit to be detected, and the corresponding induced electromotive force is obtained. The sampling resistor is connected to the PCB Rogowski coil to obtain the sampling voltage corresponding to the induced electromotive force. The signal amplification circuit is connected to the sampling resistor, which is used to amplify the sampling voltage. The integration and voltage holding circuit is connected to the signal amplifying circuit, which is used to integrate the amplified sampling voltage, and obtain the voltage signal proportional to the current signal in the circuit to be detected, and is used to maintain the maximum value of the voltage signal. The micro-controller is connected to the integration and voltage holding circuit, which is used to sample the maximum value of the voltage signal by ADC. According to the ADC sampling data and the preset current calibration data, the current peak value of the current signal in the circuit to be detected is obtained. On the one hand, the current sampling is carried out through the PCB Rogowski coil, and the non-contact detection is adopted, which will not affect the original discharge loop, and has high security, fast response speed, saturation current up to several thousand amperes, and the PCB coil has simple structure, stable process, high consistency and low cost. On the other hand, the invention can stably and accurately measure the peak current valued of each time through the signal amplification circuit and the integration and voltage holding circuit, and can monitor the treatment effect in real time when the vascular calcification treatment equipment is used for treatment.

DESCRIPTION OF THE DRAWINGS

Explanation Of The Drawings

The accompanying drawings set forth herein are intended to provide a further understanding of the present invention and constitute a part of this application, illustrative embodiments of the present invention and the description thereof are intended to explain the present invention and are not to be construed as unduly limiting the present invention.

FIG. 1 is a block diagram of a current peak value measurement apparatus according to an embodiment of the present invention;

FIG. 2 is a schematic diagram of an overall structure of a PCB Rogowski coil according to an embodiment of the present invention;

FIG. 3 shows a schematic view of the turns of the PCB Rogowski coil in area B of FIG. 2;

FIG. 4 is a schematic circuit diagram of a current peak value measurement apparatus according to an embodiment of the present invention;

FIG. 5 is a schematic diagram of a high voltage generator according to an embodiment of the present invention;

FIG. 6 is a schematic diagram of a therapeutic device for vascular calcification according to an embodiment of the present invention.

## DETAILED DESCRIPTION OF THE PRESENT APPLICATION

Optimal Embodiment Of The Invention

In order to make those skilled in the art better understand the technical solutions of the present invention, with reference to the accompanying drawings in the embodiments of the present invention, the technical solutions in the embodiments of the present invention are clearly and completely described. The described embodiments are only part of the embodiments of the present invention, but not all of the embodiments. All other embodiments obtained by those of ordinary skill in the art based on the embodiments of the present invention without creative efforts should fall within the scope of protection of the present invention.

It should be noted that, the terms "first", "second" and the like in the description, the claims and the above-mentioned figures of the invention are used to distinguish between similar objects, but need not be used to describe a particular sequence or precedence, it being understood that the data so used may be interchanged under appropriate circumstances, so that the embodiments of the invention described herein can be practiced in an order other than those illustrated or described herein. The terms "including" and "having" and any variations thereof, it is intended to cover a non-exclusive inclusion, e.g., a process, method, system, product, or apparatus that includes a series of steps or units, is not necessarily limited to those steps or units that are expressly listed, but may include other steps or units that are not expressly listed or are inherent to such process, method, product, or apparatus.

Unless otherwise defined, all technical and scientific terms used herein have the same meaning as commonly understood by one of ordinary skill in the art to which the invention pertains. The terms used in the description of the invention herein are for the purpose of describing particular embodiments only and are not intended to be limiting of the invention.

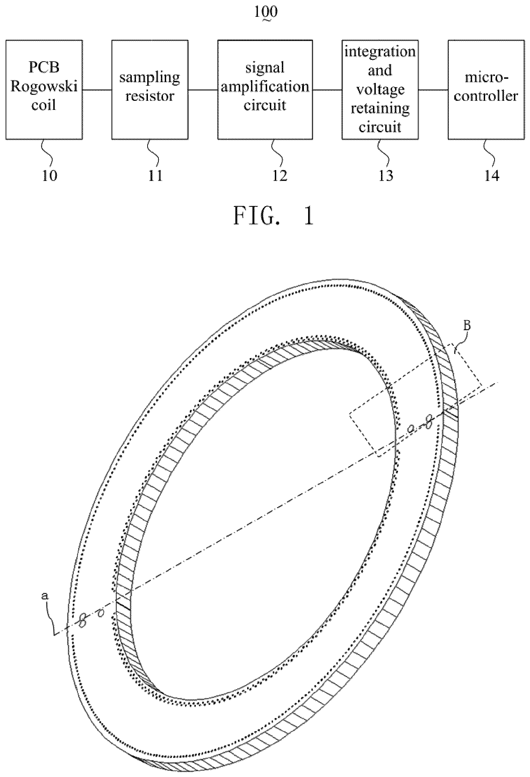

Please refer to FIG.1, FIG.1 shows a structural block diagram of a peak current value measurement apparatus provided by an embodiment of the present invention. As shown in FIG. 1, the peak current value measurement apparatus 100 includes a PCB Rogowski coil 10, a sampling resistor 11, a signal amplification circuit 12, an integration and voltage holding circuit 13 and a micro-controller 14. Wherein, the PCB Rogowski coil 10 is configured to induce a current signal in a circuit to be detected, and obtain the corresponding induced electromotive force. The sampling resistor 11 is connected to the PCB Rogowski coil 10, and is configured to obtain sampling voltage corresponding to the induced electromotive force. The signal amplifying circuit 12 is connected to the sampling resistor 11, and is configured to amplify sampling voltage and output amplified sampling voltage. The integrating and voltage holding circuit 13 is connected to the signal amplifying circuit 12, and is configured to perform integrating processing on amplified sampling voltage to obtain a voltage signal proportional to the current signal in the circuit to be detected, and to hold a maximum value of the voltage signal. The micro-controller 14 is connected to the integrating and voltage holding circuit 13 and is configured to carry out ADC sampling on the maximum value of the voltage signal and obtain a current peak value of the current signal in the circuit to be detected according to ADC sampling data and preset current calibration data.

Specifically, according to the induction principle of the PCB Rogowski coil 10, that is, when the current flowing through the conducting wire in the PCB Rogowski coil 10 is I (t), a magnetic field is generated around the current-carrying conductor, the closer the current-carrying conductor is, the stronger the magnetic field intensity is, and the magnetic field intensity at the position r away from the current-carrying conductor is:

$$H=(I(t))/2\pi r.$$

The magnetic induction here is:

$$B=(\mu_0 I(t))/2\pi r.$$

Wherein the vacuum permeability $\mu_0 = 4\pi \times 10^{-7}$ H/m, $\pi$ is the circumferential ratio.

There is a magnetic flux in the wire turns of the PCB Rogowski coil 10, and the magnetic flux of each turn is:

$$\varphi = \int B dS.$$

Wherein S is the area of single wire turn, d is the differential symbol and dS is the differential of the area. The magnetic flux is related to the magnetic induction intensity and the area of wire turn.

According to Faraday's law of electromagnetic induction, when the measured current is changed, the coil can produce induced electromotive force:

$$e(t) = -N \, d\varphi/dt = -M \, (dI(t))/dt;$$

In the formula, N is the number of wire turns, M is the mutual inductance of coils, $d\varphi$ is the variation of magnetic flux, dt is the variation of time, and dI (t) is the variation of current.

The induced electromotive force of the PCB Rogowski coil 10 is proportional to the time difference of the measured current, so that the waveform of the measured current can be restored by connecting an integrating circuit.

The induced electromotive force is related to the number of wire turns and the area of the wire turns, so that by increasing the number of wire turns and increasing the induced magnetic flux of the coil, the induced electromotive force can be increased. On the other hand, by properly increasing the width of the wire turns, the area of the wire turns can be increased to a certain extent, so that the magnetic flux passing through the hollow wire turns can be increased and the induced electromotive force can be increased.

Therefore, the present invention induces the current signal in the circuit to be detected through the PCB Rogowski coil 10, and obtains the corresponding induced electromotive force; the sampling resistor 11 is connected to the PCB Rogowski coil 10 to obtain a sampling voltage corresponding to the induced electromotive force; the signal amplifying circuit 12 is connected to the sampling resistor 11 to amplify and output the sampling voltage; the integration and voltage holding circuit 13 is connected to the signal amplification circuit 12, performs integration processing on the amplified sampling voltage to obtain a voltage signal proportional to the current signal in the circuit to be detected, and holds the maximum value of the voltage signal; the micro-controller 14 is connected to the integrating and voltage holding circuit 13, performs ADC sampling on the maximum value of the voltage signal, and obtains the current peak value of the current signal in the circuit to be measured according to the ADC sampling data and the preset current calibration data.

Wherein the micro-controller 14 may set the sampling port as an ADC input, and turn on ADC sampling. A timer may also be configured for periodically sampling the ADC signal, the timing time can be adjusted as required, and count down. When the count value reaches the set value 0, it will generate a timer interrupt, wherein the interrupt interval time is a set time. The ADC sampled data processed in an interrupt service function, after an interrupt occurs, the interrupt flag bit needs to be cleared to occur interrupt again. At the same time, the count value will be reloaded as the set count value, and counting again. After the ADC data is received again, a sampling value can be obtained. Under the condition of short sampling time interval, the current change curve can be accurately restored. Of course, it is also possible to sample only the value of corresponding stage, such as peak value current.

In order to obtain the actual current in the circuit to be detected from the ADC sampling data, current calibration needs to be carried out, that is, the reference current source is used to select the current value at a certain interval as the reference value, and the corresponding data of the current peak value measurement apparatus 100 at the current is obtained respectively. In other words, when the set current flows through the circuit to be detected, the micro-controller 14 can sample the corresponding data in this state. Therefore, the corresponding linear relationship can be obtained in several stages. After the linear relationship is obtained, the sampled data of the micro-controller 14 will correspond to the actual current of the circuit to be detected one by one, and the actual current value at this time can be calculated according to the corresponding data.

It should be explained that the current peak value measurement apparatus 100 of the present invention not only can detect the peak current value, but also can restore the complete current change curve on the circuit to be detected by setting the sampling frequency of the micro-controller 14.

Embodiments Of The Invention

Embodiments Of The Invention

In one embodiment, please refer to FIG.2. FIG.2 shows the overall structure of the PCB Rogowski coil provided by one embodiment of the present invention. The PCB Rogowski coil 10 includes a first coil part 101 and a second coil part 102 (two parts divided by a dotted line a in FIG. 2), the first coil part 101 and the second coil part 102 form an loop-shaped coil whole body, and the first coil part 101 and the second coil part 102 are detachably connected; the first coil part 101 and the second coil part 102 are both provided with conducting wires on the top layer and the bottom layer correspondingly, the corresponding conducting wires are connected through the through holes to form wire turns and are provided with return wires.

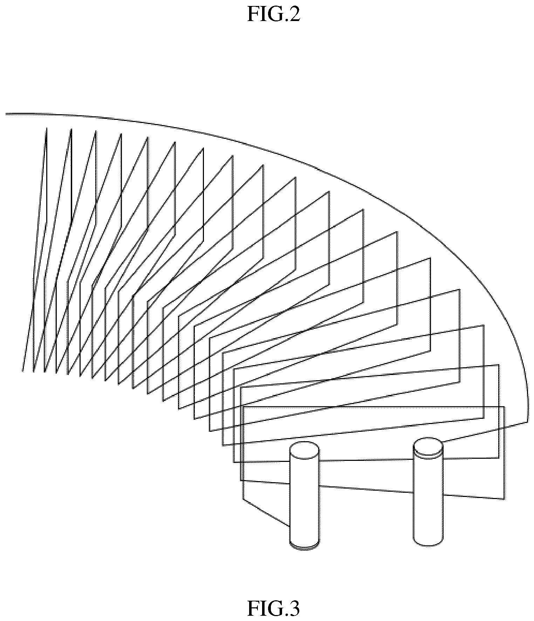

Specifically, please refer to FIG. 3, FIG. 3 shows the schematic diagram of the turns of the PCB Rogowski coil in area B of FIG. 2. As shown in FIG. 3, the top and bottom layers of the PCB are wired radially with equal spacing along the circumference, and the corresponding wires of the top layer and bottom layer are connected through the hole to form turns, that is, the top layer and bottom layer of the PCB are wired with equal spacing respectively, and then the top and bottom wires are connected through the wire to form turns, and the return line is set. There are many turns evenly distributed on the PCB, and the voltage induced by a single turn is very weak, and the multi-turn turns are in series, and the induced voltage is improved. In order to facilitate installation and removal without affecting the original line connection, the embodiments make two ring parts of PCB Rogowski coil 10, namely the first coil part 101 and the second coil part 102. Preferably, the first coil part 101 and the second coil part 102 in FIG. 2 are two semi-circle structures of the same size, which can be connected to form a complete PCB Rogowski coil 10. In actual use, the first coil coil part 101 and the second coil part 102 are connected to form a PCB Rogowski coil 10, and the line to be measured can be passed through the coil during measurement, and it can be conveniently removed after measurement if necessary.

It should be noted that, for simplicity and clarity, the specific structure of the wire turn only shows the region B in FIG.2, and other regions may extend according to the rule in FIG. 3. Meanwhile, in other embodiments, the first coil portion 101 and the second coil portion 102 may also be set to be different in size, and only the two coil portions need to be connected to form a complete PCB Rogowski coil.

In one embodiment, the current peak value measurement apparatus 100 further includes a filter circuit disposed between the PCB Rogowski coil 10 and the signal amplification circuit 12

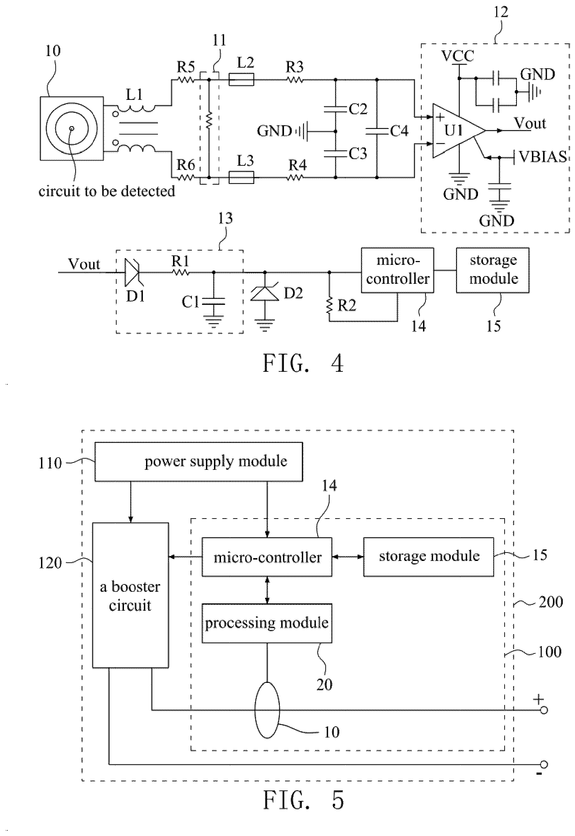

In one embodiment, please refer to FIG. 4. FIG. 4 is a schematic circuit diagram of a current peak value measurement apparatus according to an embodiment of the present invention. The signal amplification circuit 12 includes a differential amplifier U1.

Specifically, a first input terminal (+ in the figure) and a second input terminal (-in the figure) of a differential amplifier U1 differentially amplify a sampling voltage across a sampling resistor 11, and output the amplified voltage through the output terminal. Wherein the power supply terminal of the differential amplifier U1 is also connected to power supply VCC, and the bias port of the differential amplifier is connected to bias voltage VBIAS, and its power supply terminal and bias port are respectively connected to correspondent filtering unit.

In one embodiment, as showing in FIG.4, the integrating and voltage holding circuit 13 includes an RC integrating circuit and a first diode D1, wherein the RC integrating circuit includes a first resistor R1 and a first capacitor C1. The first diode D1 and the first resistor R1 are connected in series between the output terminal Vout of the signal amplifying circuit 12 and the input terminal of the micro-controller 14, and the first capacitor C1 is connected between the input terminal of the micro-controller 14 and the ground.

Specifically, the RC integrating circuit is used for integrating the output signal of the signal amplifying circuit 12, the induced electromotive force of the PCB Rogowski coil is directly proportional to the differential of the measured current to time, therefore, the waveform of the measured current can be restored only by connecting an integrating circuit. Because the diode has the unidirectional conduction characteristic, the maximum voltage value of the first capacitor C1 can be kept by arranging the first diode D1, even if the current is reduced, the maximum voltage value can also be kept, thereby ensuring that the peak value current or the current peak value of the measured current can be accurately sampled by the micro-controller 14.

In one embodiment, as shown in FIG. 4, the current peak value measurement apparatus 100 further includes a second resistor R2, wherein one terminal of the second resistor R2 is connected to the input terminal of the micro-controller 14, and the other terminal of the second resistor is connected to the control terminal of the micro-controller 14, and is used for releasing the voltage of the first capacitor C1 after sampling is completed at the input terminal of the micro-controller 14.

Specifically, after the signal at the input terminal of the micro-controller 14, i.e. the sampling port of ADC, is sampled, the voltage of the first capacitor C1 is released by controlling the port level state of the control end of the micro-controller 14, and after the voltage is released, the sampling can be carried out again.

In one embodiment, the current peak value measurement apparatus 100 further includes a second diode D2, wherein the second diode D2 is connected between the input terminal of the micro-controller 14 and ground.

Specifically, a second diode D2 is provided between the input terminal of the micro-controller 14 and the ground for preventing the voltage of the front terminal from being too high and ensuring that the voltage of the input terminal (sampling port) of the micro-controller 14 is within a stable range.

In one embodiment, the filter circuit includes a common-mode inductor L1, the common-mode inductor L1 is disposed between an output terminal of a PCB Rogowski coil 10 and a sampling resistor 11.

Specifically, the common-mode inductor L1 (Common mode Choke), also known as common mode choke, is used to suppress electromagnetic waves generated by high-speed signal lines to emit outward radiation, the common-mode inductor is essentially a bidirectional filter, on the one hand to filter out the common mode electromagnetic interference on the signal line, on the other hand, to inhibit itself from emitting electromagnetic interference, to avoid affecting the normal operation of other electronic devices in the same electromagnetic environment. That is, the common-mode electromagnetic interference on the signal line is filtered out while the suppression itself does not emit electromagnetic interference.

In one embodiment, as shown in FIG. 4, the filter circuit includes a first magnetic bead L2 and a second magnetic bead L3, the first magnetic bead L2 and the second magnetic bead L3 are connected in parallel between the sampling resistor 11 and the signal amplifying circuit 12.

Specifically, the first magnetic bead L2 and the second magnetic bead L3 are equivalent to the resistance and inductance in series, but both the resistance and inductance values vary with frequency. In the embodiments, they are used to eliminate differential mode interference, suppress high-frequency noise and peak interference, and absorb electrostatic pulses. As shown in FIG. 4, the first magnetic bead L2 is connected between the first terminal of the sampling resistance 11 and the first input terminal of the differential amplifier U1, and the second magnetic bead L3 is connected between the second terminal of the sampling resistance 11 and the second input terminal of the differential amplifier U1.

In one embodiment, a filter circuit includes a common-mode filter capacitor and a differential-mode filter capacitor disposed between the sampling resistor 11 and the signal amplification circuit 12.

In particular, as shown in FIG. 4, the common-mode filter capacitor includes a second capacitor C2 and a third capacitor C3. The second capacitor C2 is connected between the first input terminal of the differential amplifier U1 and the ground, the third capacitor C3 is connected between the second input terminal of the differential amplifier U1 and the ground. The differential-mode filter capacitor includes a fourth capacitor C4. The fourth capacitor C4 is connected between the first input terminal and the second input terminal of the differential amplifier U1. The common-mode filter capacitor and the differential-mode filter capacitor are used for filtering clutter. More accurate voltage difference can be obtained after filtering.

In one implementation mode, as shown in FIG. 4, the current peak value measurement apparatus 100 further includes a third resistor R3 and a fourth resistor R4, wherein the third resistor R3 is connected between the first magnetic bead L2 and the first input terminal of the differential amplifier U1, and the fourth resistor R4 is connected between the second magnetic bead L3 and the second input terminal of the differential amplifier U1.

In one implementation mode, as shown in FIG. 4, the current peak value measurement apparatus 100 further includes a voltage-dividing resistor, the voltage-dividing resistor includes a fifth resistor R5 and a sixth resistor R6, the fifth resistor R5 and the sixth resistor R6 are connected between the output terminal of the PCB Rogowski coil 10 and the sampling resistor 11.

In an embodiment, the micro-controller 14 is further configured to perform digital filtering on the ADC sampling data to filter abnormal data in the multiple ADC sampling data, and take an average value of the multiple ADC sampling data after filtering for obtaining a current peak value of the current signal in the circuit to be detected according to the current calibration data; wherein the abnormal data includes a minimum value and/or a maximum value of the plurality of ADC sampled data.

Specifically, if the corresponding peak current value is obtained from the single sampling value of micro-controller 14 during the preset period (there is a stable moment at the peak value of the pulse current), there may be some deviation. In order to avoid the impact of the deviation of the sampled data, the abnormal data is filtered by digital filtering in the present embodiment. That is, during data sampling, data is processed after multiple sampling times. For example, when the sampling times is 32 times, micro-controller 14 performs data processing after 32 sampling times, sorts the 32 times of data successively from smallest to largest, and removes the smallest group of data and the largest group of data after sorting. Of course, according to the time situation, it can also be the smallest multiple sets of data and the largest multiple sets of data, and then the remaining data is averaged to obtain the effective data after a filter. According to the effective data and current calibration data, the current peak value of the current signal in the circuit to be detected can be obtained.

In an embodiment, as shown in FIG.4, the current peak value measurement apparatus 100 further includes a storage module 15, wherein the storage module 15 is connected to the micro-controller 14 and is used for storing the current peak value.

Specifically, the storage module 15 may be a Flash memory, the Flash memory is a serial SPI chip, addressed according to address when data is stored. Every time the data is increased by one, the count value is automatically increased by one, the first address to be addressed is data number * data length, the count value also can be stored, the data can be stored in the address tail portion of Flash memory. When the current peak value measurement apparatus 100 is used for the therapeutic device for vascular calcification, the Flash memory also can store data information of device ID, etc. For example, the data storage format is referenced as follows:

01 02 03 04 05 00 01 0F 0A;
Among them, 01 02 03 04 05 is the ID number of the consumable;
0001 is the number of data records;

0F 0A is the current value;

The data store address is as shown in Table 1, offset on the Flash base address:

Table 1

| Storage address (hexadecimal) | Data Content | Data description |
| --- | --- | --- |
| 0x00000000 | 01 0203 04 05 00 01 0F 0A | Stored first frame data |
| 0x00000009 | 01 02 03 04 05 00 02 0D 0F | Stored second frame data |
| 0x00000012 | 01 02 03 04 05 00 03 0E 02 | Stored third frame data |
| ... | ... | ... |

In conclusion, the present invention, in one aspect, performs current sampling through a PCB Rogowski coil 10, using non-contact detection, does not affect the original discharge loop. Its advantages are high safety and response speed, and high saturation current up to thousands of amperes. Moreover, the PCB coil has the advantages of simple structure, stable process, higher consistency and lower cost; on the other hand, the peak current of each time can be stably and accurately measured through a signal amplifying circuit 12 and an integration and voltage holding circuit 13, and the treatment effect can be monitored in real time when the therapeutic device for vascular calcification is used for treatment.

The embodiment of the present invention also provides a high voltage generator, please refer to FIG.5. FIG.5 is a schematic diagram showing the structure of the high voltage generator according to an embodiment of the present invention. As shown in FIG.5, the high voltage generator 200 includes a power supply module 110, a booster circuit 120 and the current peak value measurement apparatus 100 as described in any one of the above embodiments. Wherein, the power supply module 110 is connected to the booster circuit 120 and the micro-controller 14 in the current peak value measurement apparatus 100. The micro-controller 14 in the current peak value measurement apparatus 100 is connected to the booster circuit 120. The PCB coil in the current peak value measurement apparatus 100 is arranged at the output terminal of the booster circuit 120 and is used for inducing the current signal in a circuit to be detected at the output terminal, wherein for simplicity, the signal processing module 20 in FIG.5 includes other modules, circuits and elements except the PCB Rogowski coil 10, the micro-controller 14 and the storage module 15 in the implementation mode.

Specifically, the power supply module 110 supplies power to the booster circuit 120 and the micro-controller 14, the booster circuit 120 is used for generating a high-voltage required for treatment, and the power supply voltage can be boosted to the required high-voltage through the booster circuit 120.

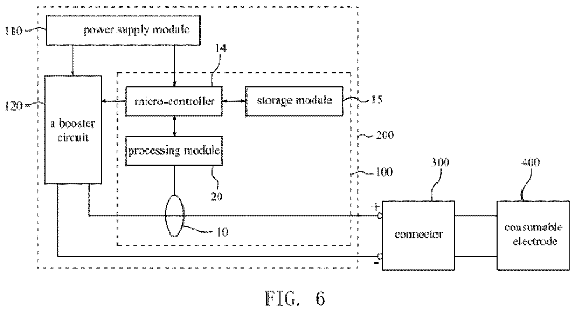

An embodiment of the present invention further provides a therapeutic device for vascular calcification, please refer to FIG.6. FIG.6 shows a schematic structural diagram of therapeutic device for vascular calcification according to an embodiment of the present invention. As shown in FIG.6, the therapeutic device for vascular calcification includes a high voltage generator, a connector 300, and a consumable electrode 400 according to the foregoing embodiments. Wherein, the output terminal of the high voltage generator is connected to the consumable electrode 400 through the connector 300 to form a high-voltage discharge loop.

Industrial Applicability

In the use of the therapeutic device for vascular calcification for the treatment of calcified tissue in blood vessels, the specific process is: through the guide wire, the internal tube with several consumable electrodes 400 distributed at the distal end and the balloon wrapped outside the internal tube are transported to the calcified tissue in the blood vessel to be treated, and the balloon is filled with liquid (conductive liquid, such as normal saline). After filling the liquid, the high voltage generator is energized, and the high voltage generator generates a high voltage discharge circuit, which makes the consumable electrode 400 produce shock wave, and through the shock wave, the calcification focus attached to the blood vessel wall is crushed.

Sequence Listing Free Content

The above is only a better embodiment of the invention and is not intended to limit the invention in any form, although the invention has been disclosed as a better embodiment, but is not intended to limit the invention, any skilled person familiar with the profession, within the scope of the technical scheme of the invention, When the technical content disclosed above is used to make some changes or modifications into equivalent embodiments of equivalent changes, but any simple modifications, equivalent changes and modifications made to the above embodiments according to the technical substance of the invention that are not removed from the content of the technical scheme of the invention, are still within the scope of the technical scheme of the invention.

**Claims**

1. A current peak value measurement apparatus is **characterized by** comprising: a PCB Rogowski coil, a sampling resistor, a signal amplification circuit, an integration and voltage holding circuit and a micro-controller; wherein,

   the PCB Rogowski coil is configured to induce a current signal in a circuit to be detected, and obtain a corresponding induced electromotive force;
   the sampling resistor is connected to the PCB Rogowski coil, and is configured to obtain sampling voltage corresponding to the induced electromotive force;
   the signal amplifying circuit is connected to the sampling resistor, and is configured to amplify the sampling voltage and output amplified sampling voltage;
   the integrating and voltage holding circuit is connected to the signal amplifying circuit, and is configured to perform integrating processing on the amplified sampling voltage to obtain a voltage signal proportional to the current signal in the circuit to be detected, and to hold a maximum value of the voltage signal;
   the micro-controller is connected to the integrating and voltage holding circuit and is configured to carry out ADC sampling on the maximum value of the voltage signal and obtain a current peak value of the current signal in the circuit to be detected according to ADC sampling data and preset current calibration data.

2. The current peak value measurement apparatus according to claim **1, characterized in that** the PCB Rogowski coil comprises a first coil part and a second coil part, the first coil part and the second coil part form a loop-shaped coil whole body, and the first coil part and the second coil part are detachably connected; wherein,
   the first coil part and the second coil part are respectively provided with conducting wires on a top layer and a bottom layer correspondingly, the corresponding conducting wires are connected through via holes to form wire turns and are provided with return wires.

3. The current peak value detecting device according to claim **1,** wherein the integrating and voltage holding circuit comprises: an RC integrating circuit and a first diode, the RC integrating circuit comprising a first resistor and a first capacitor; wherein,
   the first diode and the first resistor are serially connected between an output terminal of the signal amplifying circuit and an input terminal of the micro-controller, and the first capacitor is connected between the input terminal of the micro-controller and ground.

4. The current peak value measurement apparatus according to claim **1, characterized in that** the micro-controller is further configured to perform digital filtering on the ADC sampling data to filter abnormal data in a plurality of the ADC sampling data, and take an average value of the filtered plurality of the ADC sampling data for obtaining a current peak value of a current signal in the circuit to be detected according to the current calibration data; wherein the abnormal data comprises the minimum and/or the maximum of a plurality of the ADC sampled data.

5. The current peak value measurement apparatus according to claim **1,** further comprising a filter circuit disposed between the PCB Rogowski coil and the signal amplification circuit.

6. The current peak value measurement apparatus according to claim **5,** wherein said filter circuit comprises a common-mode inductor disposed between an output terminal of the PCB Rogowski coil and the sampling resistor.

7. The current peak value measurement apparatus according to claim **5, characterized in that** the filter circuit comprises a first magnetic bead and a second magnetic bead, the first magnetic bead and the second magnetic bead are connected in parallel between the sampling resistor and the signal amplifying circuit.

8. The current peak value measurement apparatus according to claim **5,** wherein the filter circuit comprises a common-mode filter capacitor and a differential-mode filter capacitor, the common-mode filter capacitor and the differential-mode filter capacitor are disposed between the sampling resistor and the signal amplification circuit.

9. A high voltage generator is **characterized by** comprising a power supply module, a booster circuit and a current peak value measurement apparatus as claimed in any one of claims **1** to **8;** wherein,

the power supply module is connected to the booster circuit and the micro-controller in the current peak value measurement apparatus;
the micro-controller in the current peak value measurement apparatus, is connected to the booster circuit;
the PCB coil in the current peak value measurement apparatus is arranged at an output terminal of the booster circuit and is configured to induce the current signal in the circuit to be detected at the output terminal.

10. A therapeutic device for vascular calcification is **characterized by** comprising the high voltage generator as claimed in claim **9,** a connector and a consumable electrode; wherein an output terminal of the high voltage generator is connected to the consumable electrode through the connector to form a high-voltage discharge loop.

EP 4 397 254 A1

100

| PCB Rogowski coil | sampling resistor | signal amplification circuit | integration and voltage retaining circuit | micro-controller |
|---|---|---|---|---|
| 10 | 11 | 12 | 13 | 14 |

FIG. 1

12

FIG.2

FIG.3

10

R5  11  L2  R3

L1

circuit to be detected

R6  L3  R4

GND

C2

C3

C4

12

VCC  GND

U1  +  Vout
    −  VBIAS

GND

GND

13

Vout  D1  R1

C1  D2

R2

micro-controller

14

storage module

15

FIG. 4

110  power supply module

14

120  a booster circuit  micro-controller  storage module  15

200

100

processing module  20

10

+

−

FIG. 5

14

FIG. 6

## INTERNATIONAL SEARCH REPORT

| International application No. |
| --- |
| **PCT/CN2022/115995** |

**A. CLASSIFICATION OF SUBJECT MATTER**

A61B 17/22(2006.01)i; A61B 5/24(2021.01)i; G01R 19/165(2006.01)i

According to International Patent Classification (IPC) or to both national classification and IPC

**B. FIELDS SEARCHED**

Minimum documentation searched (classification system followed by classification symbols)

A61B; G01R; A61M; A61N

Documentation searched other than minimum documentation to the extent that such documents are included in the fields searched

Electronic data base consulted during the international search (name of data base and, where practicable, search terms used)

CNTXT, ENTXTC, ENTXT, VEN, CJFD: 朴芃医疗科技, 李闯, 唐智皇, 感应, 电流, 检, 测, 峰, 最, 极, 线圈, 罗氏, 罗戈夫斯基线圈, 积分, 采样, 放大, 滤波; induce, induction, coil, resistance, current, faradic, detect+, measure, maximum, max, peak, Rogowski, integral, sample, magnify, filter

**C. DOCUMENTS CONSIDERED TO BE RELEVANT**

| Category* | Citation of document, with indication, where appropriate, of the relevant passages | Relevant to claim No. |
| --- | --- | --- |
| PX | CN 113520521 A (SHANGHAI PAF MEDTECH CO., LTD.) 22 October 2021 (2021-10-22) claims 1-10 | 1-10 |
| Y | CN 104155499 A (STATE GRID CORPORATION OF CHINA et al.) 19 November 2014 (2014-11-19) description, paragraphs 35-82, and figures 1-6 | 1-10 |
| Y | CN 112147476 A (HANGZHOU KELIN ELECTRIC CO., LTD.) 29 December 2020 (2020-12-29) description, paragraphs 22-40, and figures 1-4 | 1-10 |
| Y | CN 112674838 A (SUZHOU ZHONGHUI MEDICAL TECHNOLOGY CO., LTD.) 20 April 2021 (2021-04-20) description, paragraphs 52-119, and figures 1-7 | 9-10 |
| A | CN 101551416 A (GENERAL ELECTRIC CO.) 07 October 2009 (2009-10-07) entire document | 1-10 |
| A | CN 111157936 A (XI'AN AEROCOMM MEASUREMENT AND CONTROL TECHNOLOGY CO., LTD.) 15 May 2020 (2020-05-15) entire document | 1-10 |

☑ Further documents are listed in the continuation of Box C.　　☑ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **07 November 2022** | **21 November 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/115995**

C.     DOCUMENTS CONSIDERED TO BE RELEVANT

| Category* | Citation of document, with indication, where appropriate, of the relevant passages | Relevant to claim No. |
|---|---|---|
| A | CN 103876825 A (COVIDIEN LP) 25 June 2014 (2014-06-25)<br>entire document | 1-10 |
| A | CN 102445588 A (NAVAL UNIVERSITY OF ENGINEERING OF PLA) 09 May 2012 (2012-05-09)<br>entire document | 1-10 |
| A | US 2018074098 A1 (DELTA ELECTRONICS, INC.) 15 March 2018 (2018-03-15)<br>entire document | 1-10 |
| A | US 2010013460 A1 (SIEMENS AKTIENGESELLSCHAFT) 21 January 2010 (2010-01-21)<br>entire document | 1-10 |

Form PCT/ISA/210 (second sheet) (January 2015)

**INTERNATIONAL SEARCH REPORT**
**Information on patent family members**

International application No.

**PCT/CN2022/115995**

| Patent document cited in search report | | | Publication date (day/month/year) | Patent family member(s) | | | Publication date (day/month/year) |
|---|---|---|---|---|---|---|---|
| CN | 113520521 | A | 22 October 2021 | None | | | |
| CN | 104155499 | A | 19 November 2014 | None | | | |
| CN | 112147476 | A | 29 December 2020 | None | | | |
| CN | 112674838 | A | 20 April 2021 | None | | | |
| CN | 101551416 | A | 07 October 2009 | JP | 2009250976 | A | 29 October 2009 |
| | | | | EP | 2107381 | A2 | 07 October 2009 |
| | | | | US | 2009243590 | A1 | 01 October 2009 |
| | | | | CA | 2660173 | A1 | 01 October 2009 |
| CN | 111157936 | A | 15 May 2020 | None | | | |
| CN | 103876825 | A | 25 June 2014 | AU | 2013273611 | A1 | 03 July 2014 |
| | | | | US | 2014171935 | A1 | 19 June 2014 |
| | | | | CN | 203662903 | U | 25 June 2014 |
| | | | | CA | 2836605 | A1 | 17 June 2014 |
| | | | | EP | 2742888 | A1 | 18 June 2014 |
| CN | 102445588 | A | 09 May 2012 | None | | | |
| US | 2018074098 | A1 | 15 March 2018 | TW | 201809688 | A | 16 March 2018 |
| | | | | JP | 2018044947 | A | 22 March 2018 |
| | | | | EP | 3296753 | A1 | 21 March 2018 |
| US | 2010013460 | A1 | 21 January 2010 | EP | 2092352 | A1 | 26 August 2009 |
| | | | | MX | 2009006676 | A | 30 June 2009 |
| | | | | DE | 102006061923 | A1 | 03 July 2008 |
| | | | | WO | 2008077798 | A1 | 03 July 2008 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111005958